# EUROPÄISCHE PATENTSCHRIFT

(11) **EP 2 688 523 B1**
(45) Veröffentlichungstag und Bekanntmachung des Hinweises auf die Patenterteilung: **24.08.2016**
(21) Anmeldenummer: 12712942.7
(22) Anmeldetag: 23.03.2012
(51) Int. Cl.: A61F 2/72, A61F 2/78, A61F 2/80

(54) **PROTHESENSYSTEM**
PROSTHETIC SYSTEM
SYSTÈME DE PROTHÈSE

(30) Priorität: 25.03.2011 DE 102011015502
(43) Veröffentlichungstag der Anmeldung: 29.01.2014
(73) Patentinhaber: Otto Bock HealthCare GmbH, 37115 Duderstadt (DE)
(72) Erfinder: BERTELS, Thomas, 37115 Duderstadt (DE); GARUS, Bernard, 37574 Einbeck (DE); KETTWIG, Thomas, 37085 Göttingen (DE)
(74) Vertreter: Friedrich, Andreas
(86) Internationale Anmeldenummer: PCT/EP2012/001280
(87) Internationale Veröffentlichungsnummer: WO 2012/130416

(56) Entgegenhaltungen:
- US-A- 5 341 813
- US-A- 5 443 525

## Beschreibung

Die Erfindung betrifft ein Prothesensystem mit einem Liner aus einem elektrisch nicht leitenden Liner-Material, der vorgesehen ist, über einen Amputationsstumpf gezogen zu werden, und der eine zur Anlage an dem Amputationsstumpf vorgesehene Innenfläche und eine der Innenfläche abgewandte Außenfläche aufweist, wenigstens einer Elektrode, die wenigstens eine Kontaktfläche aufweist, und einem Prothesenschaft, der vorgesehen ist, an dem Amputationsstumpf angeordnet zu werden, nachdem über diesen der Liner gezogen wurde, so dass eine Schaftinnenfläche der Außenfläche des Liners zugewandt ist. Die Erfindung betrifft zudem einen Liner und einen Prothesenschaft für ein derartiges Prothesensystem.

Bei einem herkömmlichen Prothesensystem der hier angesprochenen Art bildet der Prothesenschaft einen Teil, der den amputierten Teil einer Extremität eines Patienten ersetzt. Der Liner hat unter anderem die Aufgabe, eine polsternde und sich an den Amputationsstumpf anpassende oder angepasste Zwischenschicht zwischen dem Stumpf und der Innenwand des Prothesenschaftes zu bilden.

Aus dem Stand der Technik ist es bekannt, über wenigstens eine Elektrode myoelektrische Signale aufzunehmen. Dazu wird die Elektrode an der Haut des Amputationsstumpfes angeordnet. Durch Muskelkontraktionen im Amputationsstumpf können myoelektrische Elektroden elektrische Muskelkontraktionssignale abnehmen, durch die die Steuerung entsprechender Prothesenfunktionen möglich wird. So ist es beispielsweise möglich, bei einer Unterarm- und Handprothese Funktionen der Hand über diese aus Muskelkontraktionen im Amputationsstumpf entstehenden elektrischen Signale zu steuern. Insbesondere für Arm- und Handprothesen ist die myoelektrische Steuerung im Stand der Technik bekannt, sie kann jedoch auch für Bein- und Fußprothesen verwendet werden.

Es kann zudem sinnvoll sein, einen Oberflächenwiderstand der Haut des Amputationsstumpfes elektrisch zu bestimmen, indem beispielsweise ein Stromfluss zwischen zwei oder mehr Elektroden oder Elektrodenabschnitten gemessen wird. So kann beispielsweise bestimmt werden, ob die Haut innerhalb des Liners transpiriert, wodurch sich der Sitz des Liners auf dem Amputationsstumpf und damit der Sitz des Prothesensystems verschlechtern kann.

Umgekehrt kann es sinnvoll sein, elektrische Signale auf die Haut des Amputationsstumpfes zu übertragen, um beispielsweise Muskelkontraktionen im Amputationsstumpf anzuregen.

Herkömmlicherweise befindet sich die wenigstens eine Elektrode direkt im Liner des Prothesensystems, da hier die einzige Stelle ist, mit der das Prothesensystem und damit auch die Elektrode mit der Haut des Amputationsstumpfes in Kontakt kommen kann. Wenn myoelektrische Signale abgenommen werden sollen, ist eine exakte Positionierung der Elektrode relativ zu dem Muskel, der die myoelektrischen Signale generiert, extrem wichtig. Eine nur leichte Verschiebung der Elektrode auf dem Amputationsstumpf hat bereits zur Folge, dass zwar immer noch myoelektrische Signale abgenommen werden können, die sich jedoch von den myoelektrischen Signalen unterscheiden, die am richtigen Ort am Amputationsstumpf von der Elektrode abgenommen werden können. Eine Steuerung in dem Prothesensystem, die die abgenommenen elektrischen Signale weiterverarbeitet und zur Steuerung der Prothesenfunktionen verwendet, erkennt die von einer verschobenen Elektrode abgenommenen Signale gegebenenfalls nicht, so dass es zu Fehlfunktionen und Funktionsausfällen des Prothesensystems kommen kann.

Ein Liner besteht herkömmlicherweise aus einem nicht leitenden Material, beispielsweise einem Elastomer, einem textilbeschichteten Elastomer oder einem 3D-Textil. Das Anlegen des Liners an den Amputationsstumpf erfolgt oftmals ähnlich wie das Anziehen eines Strumpfes. Der Liner, an dem herkömmlicherweise die Elektrode angeordnet ist, wird zum Anlegen in der Regel aufgerollt und im aufgerollten Zustand an die Spitze des Amputationsstumpfes angelegt. Anschließend wird der Liner wieder abgerollt und so, ähnlich wie ein Strumpf, über den Amputationsstumpf gezogen. Dabei kann es leicht zu einer Verdrehung oder Verschiebung des Liners um einige Grad kommen. Dadurch, dass der Liner, beispielsweise aus Silikon, eng am Amputationsstumpf anliegt, ist eine spätere Korrektur der Position des Liners relativ zum Amputationsstumpf nur schwer und für den Patienten unangenehm und mit großen Mühen durchzuführen.

Befindet sich an dem Liner die Elektrode, wie dies aus dem Stand der Technik bekannt ist, hat eine leichte Verschiebung des Liners auch eine leichte Verschiebung der Elektrode zur Folge, wodurch die von der Elektrode abgenommenen myoelektrischen Signale nicht mehr die von der Steuerung des Prothesensystems erwarteten Signale sind, so dass es zu den bereits angesprochenen Fehlfunktionen oder Funktionsausfällen des Prothesensystems kommen kann.

Um eine exaktere Positionierung der Elektrode relativ zum Amputationsstumpf zu gewährleisten, ist es bekannt, im Liner eine Ausnehmung, ein so genanntes Fenster, vorzusehen, dessen Ausmaße größer sind als die zu positionierende Elektrode. Die Elektrode wird entweder separat befestigt oder ist am Prothesenschaft angeordnet, der relativ zum Amputationsstumpf viel leichter zu positionieren ist. Dadurch, dass das Fenster größere Ausmaße hat als die zu positionierende Elektrode, ist auch eine leichte Verschiebung des Liners, und damit des Fensters, unschädlich. Die Elektrode wird immer noch im Fenster des Liners positioniert. Nachteilig ist jedoch, dass zwischen der Elektrode und dem Liner ein Zwischenraum entsteht, der nicht vom Liner oder der Elektrode abgedeckt wird. In diesem Zwischenraum kann die Haut eingedrückt werden, so dass es zu so genannten Fensterödemen kommen kann.

Aus dem Stand der Technik ist zudem bekannt, dass für jeden Patienten ein kostenintensiver, individueller Liner angefertigt wird, der genau auf die Form des Amputationsstumpfes angepasst wird. Dadurch ist auch eine exakte Positionierung des Liners und damit der an diesem angeordneten Elektrode relativ zum Amputationsstumpf möglich. Nachteilig ist jedoch, dass die Herstellung nicht nur kostenintensiv, sondern auch zeitaufwändig ist, so dass der Patient gegebenenfalls wochenlang auf sein Prothesensystem warten muss.

Aus der US 5,443,525, die den nächstliegenden Stand der Technik darstellt, ist ein Liner bekannt, der zur Aufnahme myoelektrischer Elektroden vorgesehen ist. Hierzu wird in ein Fenster des Liners ein flexibles weiches Polster eingeklebt, in das Elektroden eingearbeitet sind. Die Elektrodenanordnung ist somit über das Polster auf die Innenseite des Liners geklebt und durch das Fenster des Liners zugänglich, so dass die von den Elektroden abgenommenen myoelektrischen Signale weitergeleitet werden können. Nachteilig ist, dass auch bei diesem Ausführungsbeispiel eine exakte Positionierung der Elektroden relativ zum Amputationsstumpf nur über eine exakte Positionierung des Liners erreicht wird. Auch hier ist also entweder ein aufwändiges und für den Patienten unangenehmes Anlegeverfahren des Liners nötig oder eine kostenintensive und zeitaufwändige Herstellung eines individuellen, angepassten Liners erforderlich. Zudem ist die Anordnung aufwändig herzustellen und weist nur einen begrenzten Tragekomfort auf. Das Fenster des Liners bedarf zudem eines besonderen Abdichtungsaufwandes, wenn, wie häufig üblich, der Liner luftdicht ausgebildet sein muss, um mit Hilfe eines im Innenraum des Liners ausgebildeten Unterdrucks den Liner am Amputationsstumpf zu halten.

Aus der US 5,341,813 A ist eine Anordnung bekannt, die über mehrere Elektroden verfügt, die an einem Amputationsstumpf eines Patienten angeordnet werden, um die Muskeltätigkeit in diesem Amputationsstumpf testen zu können. Dazu nehmen die Elektroden Signale auf, die weiter bearbeitet und an einer Anzeigeeinheit angezeigt werden. Aus den so angezeigten Daten werden Positionen bestimmt, an denen bei einer anzufertigenden Prothese myoelektrische Elektroden anzuordnen sind.

Aus der US 2009/0216339 A1 ist ein System bekannt, mit dem myoelektrische Signale durch einen Liner hindurch geleitet werden können. Dabei werden Einsätze in den Liner eingeklebt, die einen ersten Teil aufweisen, der mit der Haut des Patienten in Kontakt kommen, einen zweiten Teil, der durch den Liner hindurchführt, und einen dritten Teil, der an der Außenseite des Liners angeordnet ist. Dieser dritte Teil weist dabei eine besonders große Oberfläche auf, um zu gewährleisten, dass eine Elektrode mit einer Kontaktfläche besonders leicht und sicher mit dieser Fläche in Kontakt gebracht wird. Auch hier ist jedoch nachteilig, dass zwar eine Positionierung der Elektrode relativ zum Liner nicht besonders genau erfolgen muss, da die Einsätze im dritten Teil eine große Fläche aufweisen, die Positionierung des Liners relativ zum Amputationsstumpf jedoch exakt erfolgen muss, damit die myoelektrischen Signale an der richtigen Stelle abgenommen werden können. Auch hier ist folglich eine Herstellung eines individualisierten, angepassten Liners notwendig.

Aus der DE 10 2007 035 409 ist ein Liner aus einem 3D-Textil bekannt, der Elektroden auf der Unterseite aufweist. Diese können auch in dem 3D-Textil eingearbeitet sein. Wie die elektrische Durchkontaktierung stattfindet, ist jedoch nicht offenbart.

Der Erfindung liegt somit die Aufgabe zugrunde, ein gattungsgemäßes Prothesensystem so weiterzuentwickeln, dass die Abnahme elektrischer, insbesondere myoelektrischer Signale an der richtigen Stelle einfach, kostengünstig und für den Patienten komfortabel möglich ist.

Die Erfindung löst die gestellte Aufgabe durch ein gattungsgemäßes Prothesensystem, die sich dadurch auszeichnet, dass die wenigstens eine Elektrode an der Außenfläche des Liners oder an der Schaftinnenfläche des Prothesenschaftes angeordnet ist, dass in wenigstens einem Bereich des Liners eine Mehrzahl von Durchleitungen von der Innenfläche zu der Außenfläche aus einem elektrisch leitenden Material in dem Linermaterial angeordnet sind, und dass die wenigstens eine Kontaktfläche der wenigstens einen Elektrode vorgesehen ist, mit wenigstens einer Durchleitung in Kontakt zu kommen, wenn der Prothesenschaft an dem Amputationsstumpf angeordnet wird, nachdem über diesen der Liner gezogen wurde.

Dadurch, dass die wenigstens eine Elektrode an der Außenfläche des Liners oder an der Schaftinnenfläche des Prothesenschaftes angeordnet ist, ist eine exakte Positionierung der Elektrode relativ zum Amputationsstumpf einfach und bequem möglich.

Der Liner ist in wenigstens einem Bereich mit einer Mehrzahl von Durchleitungen ausgestattet, die voneinander elektrisch isoliert sind. Jede dieser Durchleitungen weist eine Fläche an der Innenseite des Liners und eine Fläche an der Außenseite des Liners auf. Durch diese Durchleitungen ist es möglich, an der jeweiligen Stelle der Durchleitung elektrische Signale vom Amputationsstumpf abzunehmen und durch den Liner hindurchzuleiten. Wenn über einen Amputationsstumpf zunächst der Liner gezogen wird und über diesen der Prothesenschaft angeordnet wird, kommt die Elektrode, die an der Innenseite des Prothesenschaftes angeordnet ist, mit wenigstens einer dieser Durchleitungen in Kontakt. Auf diese Weise kann die Elektrode die elektrischen Signale von der Haut des Amputationsstumpfes durch den Liner hindurch abnehmen. Der Ort dieser Abnahme wird dabei lediglich dadurch bestimmt, welche der Durchleitungen mit der Kontaktfläche der wenigstens einen Elektrode in Kontakt kommt. Somit wird die Position, an der die elektrischen Signale abgenommen werden, im Wesentlichen durch die Position der Elektrode bestimmt. Eine geringe Verschiebung oder Verdrehung des Liners relativ zum Amputationsstumpf ist daher für die Position der Signalabnahme unschädlich.

Erfindungsgemäß ist der wenigstens eine Bereich, in dem der Liner mit einer Mehrzahl von Durchleitungen versehen ist, größer als, bevorzugt wenigstens doppelt so groß wie die wenigstens eine Kontaktfläche der wenigstens einen Elektrode, so dass ein Verdrehen oder Verschieben des Liners relativ zu dem Amputationsstumpf für die Qualität abgenommener elektrischer Signale unschädlich ist. Auf diese Weise ist sichergestellt, dass auch bei größerer Verdrehung oder Verschiebung des Liners relativ zum Amputationsstumpf und damit auch relativ zum Prothesenschaft die wenigstens eine Kontaktfläche der wenigstens einen Elektrode noch immer mit wenigstens einer der Durchleitungen in Kontakt kommt.

Als vorteilhaft hat sich herausgestellt, wenn mehrere Elektroden vorgesehen sind, die an der Schaftinnenfläche des Prothesenschaftes angeordnet sind und der Liner mehrere Bereiche mit einer Mehrzahl an Durchleitungen aufweist. Auf diese Weise ist die Abnahme von elektrischen, insbesondere myoelektrischen, Signalen an mehreren Stellen des Amputationsstumpfes möglich, wodurch eine größere Anzahl unterschiedlicher Signale und damit unterschiedlicher Steuerbefehle für die Prothesenfunktionen abgenommen werden können. Damit sind Prothesen möglich, die wesentlich mehr und komplexere Funktionen aufweisen können, so dass sie dem zu ersetzenden Körperteil ähnlicher sind.

Vorteilhafterweise ist dabei jeder Position der Elektrode an der Schaftinnenseite des Prothesenschaftes ein Bereich des Liners zugeordnet, in dem dieser mit der Mehrzahl von Durchleitungen ausgestattet ist. Alternativ dazu kann natürlich auch der Liner über seine gesamte Fläche mit einer Mehrzahl von Durchlässen ausgestattet sein. Dies ist jedoch in den meisten Fällen aus wirtschaftlichen Gesichtspunkten nachteilig.

Vorzugsweise ist die Mehrzahl von Durchleitungen einstückig mit dem Liner ausgebildet. Dies kann beispielsweise dadurch erreicht werden, dass in das Linermaterial, das vorzugsweise ein Polymerisat, beispielsweise Silikon, ist, ein leitender Abschnitt beispielsweise vor dem Auspolymerisieren eingesetzt worden ist. Beim Auspolymerisieren des Materials des Liners wird der Liner mit dem leitenden Abschnitt zu einem einheitlichen Teil verbunden, so dass sich ein einstückiger Liner ausbildet. Alternativ dazu kann die Mehrzahl an Durchleitungen auch durch in das Linermaterial eingebrachte Nieten oder Verschraubungen ausgebildet werden. Diese Nieten beziehungsweise Verschraubungen können beispielsweise aus Kupfer, Titan oder einem leitfähigen Kunststoff bestehen. Alternativ ist es des Weiteren möglich, durch den Liner hindurch einen leitfähigen Kunststoff zu spritzen. Bei einem Liner aus einem 3D-Textil werden hierzu beispielsweise Düsen durch den Stoff geführt. Bei einem Liner aus einem Polymer-Material wird das Material durchstoßen oder gelocht und die Düsen werden durch die so entstandenen Löcher geführt. Die Düsen können auch selbst ausgebildet sein, das Material zu durchstoßen. Durch die Düsen wird dann der leitfähige Kunststoff eingebracht.

In allen Fällen ist es vorteilhaft, wenn jede der Mehrzahl an Durchleitungen ein elektrischer Leiter ist, der senkrecht zur Innenfläche des Liners und damit auch senkrecht zur Haut des Patienten am Amputationsstumpf steht. Auf diese Weise entspricht die Position der Abnahme der elektrischen Signale der Position der jeweiligen Durchleitung relativ zum Amputationsstumpf und damit auch der Position der Elektrode. Da diese sehr einfach relativ zum Amputationsstumpf positionierbar ist, ist eine Verschiebung, Verdrehung oder Verrückung des Liners relativ zum Amputationsstumpf in dieser Ausgestaltung besonders unschädlich.

Vorteilhafterweise ist die wenigstens eine Elektrode verschieblich an der Außenseite des Liners oder an der Schaftinnenfläche angeordnet. Auf diese Weise kann auch nach dem Anpassen des Prothesenschaftes an den Amputationsstumpf noch die Position der Elektrode relativ zum Amputationsstumpf nachjustiert werden, um hier die optimale Position zu erreichen.

Vorzugsweise weist die wenigstens eine Elektrode mehrere Kontaktflächen auf. Auch auf diese Weise ist es möglich, eine größere Anzahl von elektrischen Signalen abzunehmen und so komplexere Funktionen und Steuersignale für das Prothesensystems zu erreichen.

In einer bevorzugten Ausführungsform ist eine Fläche jeder Durchleitung an der Außenseite für den Kontakt mit der wenigstens einen Kontaktfläche der wenigstens einen Elektrode größer oder gleich einem Quadratmillimeter. Diese Größe ist ausreichend, um eine sichere Kontaktierung zu gewährleisten. Insbesondere kann eine permanente Kontaktierung über den Druck der Elektrode an den Liner gewährleistet sein. Gleichzeitig ist die Fläche klein genug, um zwei benachbarte Elektroden so weit voneinander zu beabstanden, dass sie elektrisch gegeneinander isoliert sind. Damit wird ein Kurzschluss vermieden und die Funktionsfähigkeit aufrechterhalten.

Ein erfindungsgemäßer Liner für ein oben beschriebenes Prothesensystem zeichnet sich insbesondere dadurch aus, dass eine Fläche jeder Durchleitung an der Innenfläche des Liners genauso groß ist wie die Fläche jeder Durchleitung an der Außenfläche des Liners.

Ein erfindungsgemäßer Prothesenschaft kann in einem oben beschriebenen Prothesensystem verwendet werden und zeichnet sich insbesondere dadurch aus, dass die wenigstens eine Elektrode an der Schaftinnenseite angeordnet ist. Dabei kann an der Schaftinnenseite für die anzuordnende Elektrode durchaus auch eine Ausnehmung vorhanden sein. Die Formulierung, dass die Elektrode an der Schaftinnenseite befestigt oder angeordnet ist, bedeutet im Sinne dieser Erfindung insbesondere, dass die Elektrode in Kontakt mit den Durchleitungen treten kann, die im Liner vorgesehen sind.

Mit einem erfindungsgemäßen Prothesensystem ist es möglich, dass für eine myoelektrische Versorgung eines Prothesensystems keine individuelle Anfertigung eines Liners mehr nötig ist, sodass dieser in Standardgrößen vorproduziert werden kann und/oder der Liner dergestalt ausgelegt ist, dass der Liner sich über die Elastizität an dem Stumpf anformt und so auch Zwischengrößen abgedeckt werden. Auch muss dieser Standardliner nicht zwingend passgenau und exakt ausgerichtet angezogen werden. Eine Verdrehung oder Verschiebung des Liners um einige Winkelgrade ist durchaus möglich und für die Qualität der abgenommenen elektrischen Signale unschädlich. Durch die Anordnung der Mehrzahl von Durchleitungen ist immer ein elektrischer Kontakt mit der Kontaktfläche der wenigstens einen Elektrode gegeben. Die Bereiche, in denen der Liner mit der Mehrzahl von Durchleitungen versehen ist, sind vorteilhafterweise so groß, dass der Liner insbesondere in der Länge individuell gekürzt werden kann, ohne dass ein vollständiger dieser Bereiche abgetrennt wird. So kann der Liner mit einfachen Mitteln zumindest etwas individuell angepasst werden, ohne dass die Qualität der abgenommenen myoelektrischen Signale beeinträchtigt wird. Zudem wird der Liner bis auf das Ablängen nicht zusätzlich beschädigt.

Ein weiterer Vorteil besteht darin, dass ein Orthopädie-Techniker, der einen oben beschriebenen Liner als Standardliner verwenden möchte, nicht auf eine Elektrodenposition an der Schaftinnenseite des Prothesenschaftes festgelegt ist. Die Position der Elektroden ist hier in gewissen Grenzen frei wählbar, so dass die für den Patienten optimale Position ausgewählt werden kann.

In einer bevorzugten Ausgestaltung wird das Prothesensystem atmungsaktiv ausgestaltet. Der Liner ist dann ein 3D-Abstandsgewirk, durch das beispielsweise Nieten oder ein leitfähiger Kunststoff als elektrische Kontakte hindurchgeführt werden. Dies kann aufgrund der Textilstruktur des Liners besonders einfach geschehen. Der Prothesenschaft, insbesondere ein Innenschaft, fasst das Volumen und sorgt für die gewünschte Orientierung der Elektrode am Körper. Der Innenschaft weist dabei bevorzugt Öffnungen auf, um die Atmung, also die Luftzirkulation durch diese Öffnungen, zu gewährleisten.

In einer bevorzugten Ausgestaltung eines Prothesensystems ist das nicht leitende Linermaterial ein hydrophobes Material. Alternativ oder zusätzlich dazu ist vorteilhafterweise das elektrisch leitende Material der Durchleitungen ein hydrophiles Material.

Um einen guten elektrischen Kontakt zwischen den Durchleitungen an der Innenseite des Liners und der Haut des Trägers des Prothesensystems zu erreichen, ist es vorteilhaft, wenn die Kontaktstelle angefeuchtet ist. Dies ist mit Durchleitungen aus einem hydrophilen elektrisch leitenden Material besonders einfach möglich. Wird gleichzeitig ein hydrophobes Material als Linermaterial verwendet, kann auf diese Weise ein besonders einfach und reproduzierbar anlegbarer Liner und damit ein Prothesensystem erreicht werden. So ist es beispielsweise möglich, den Liner vor dem Anlegen zu benetzen, beispielsweise mit Wasser zu füllen. Anschließend wird das Wasser aus dem Liner entfernt, beispielsweise ausgekippt. Das hydrophobe, also wasserabweisende, Material des Liners ist danach nahezu vollständig trocken, während das hydrophile, also wasseranziehende, Material der Durchleitungen weiter angefeuchtet bleibt. Auf diese Weise ist genau der Teil der Innenseite des Liners feucht, der für eine gute elektrische Kontaktierung nötig ist, während der verbleibende Rest trocken ist, so dass hier ein angenehmes sauberes Tragegefühl entsteht.

Zudem lässt sich ein derartiger Liner besonders einfach reinigen und die Hautverträglichkeit wird erhöht.

Insbesondere wir durch die hydrophobe und hydrophile Eigenschaft der entsprechenden Flächen an der Liner-Innenseite erreicht, dass zumindest nahezu keine Restflüssigkeit an den hydrophoben Anteilen vorhanden ist, so dass die Gefahr von Kurzschlüssen und ungewollten Übertragungen elektrischer Signale verringert oder sogar vollständig beseitigt wird. Dadurch wird eine sauberere Signalübertragung möglich.

Natürlich können aktive und passive Elektroden, also mit integriertem oder separatem Verstärkerelement, verwendet werden.

Alternativ zu einem von sich aus hydrophilen oder hydrophoben Material kann natürlich auch eine in dem entsprechenden Bereich vorgesehene hydrophile oder hydrophobe Beschichtung vorgesehen sein. Dies bedeutet, die Innenseite des Liners, an der keine Durchleitungen vorgesehen sind, kann mit einem hydrophoben Material beschichtet sein. Alternativ oder zusätzlich dazu kann die Innenseite des Liners in dem Bereich, in dem die Durchleitungen vorgesehen sind, oder gegebenenfalls nur die Innenseite der Durchleitungen, mit einem hydrophilen Material beschichtet sein.

Die Hydrophobie von Materialien kann beispielsweise durch den Kontaktwinkel angegeben werden. Je größer der Kontaktwinkel ist, desto hydrophober ist die jeweilige Oberfläche. Dabei werden Oberflächen mit einem Kontaktwinkel von weniger als 90° als hydrophil und Materialien mit einem Kontaktwinkel von mehr als 90° als hydrophob bezeichnet.
Mit Hilfe einer Zeichnung wird nachfolgend ein Ausführungsbeispiel der vorliegenden Erfindung näher erläutert. Es zeigt
- Figur 1: einen Liner mit einer Mehrzahl von Durchleitungen für ein Prothesensystem gemäß einem Ausführungsbeispiel der vorliegenden Erfindung,
- Figur 2: die schematische Darstellung einer Elektrode für ein Prothesensystem gemäß einem Ausführungsbeispiel der vorliegenden Erfindung,
- Figur 3: die Anordnung einer Mehrzahl von Durchleitungen mit einer vergrößerten Darstellung einer Durchleitung für ein Prothesensystem gemäß einem weiteren Ausführungsbeispiel der vorliegenden Erfindung,
- Figur 4: die schematische Darstellung eines Liners sowie einer Elektrode für ein Prothesensystem gemäß einem weiteren Ausführungsbeispiel der vorliegenden Erfindung,
- Figur 5a, 5b: die Anordnung einer Elektrode relativ zu einer Mehrzahl von Durchleitungen,
- Figur 6a, b und c: die schematische Darstellung einer Elektrode in Kontakt mit einer Mehrzahl von Durchleitungen in einer Seitenansicht und
- Figur 7: die schematische Darstellung eines Prothesensystems gemäß einem Ausführungsbeispiel der vorliegenden Erfindung.

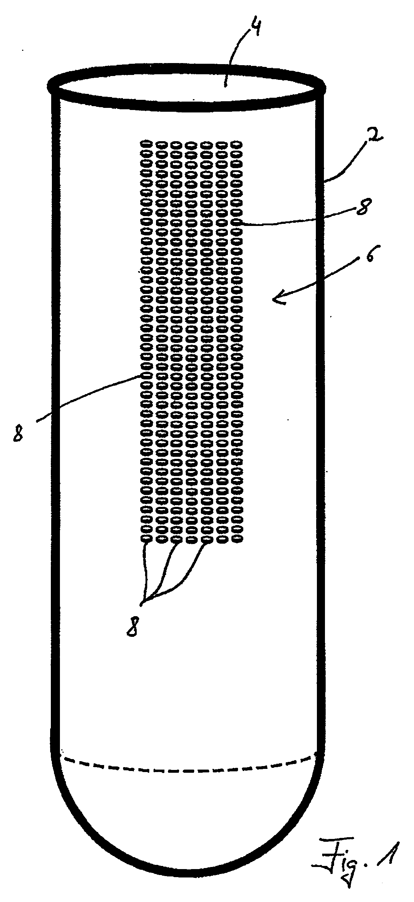

Figur 1 zeigt einen Liner 2 für ein Prothesensystem gemäß einem Ausführungsbeispiel der vorliegenden Erfindung. Der Liner 2 besteht aus einem elektrisch nicht leitenden Linermaterial, das beispielsweise Silikon sein kann. An der in Figur 1 oberen Seite des Liners 2 befindet sich eine Öffnung 4, in die der Amputationsstumpf eingeführt wird. Am Liner 2 befindet sich ein Bereich 6, in dem eine Mehrzahl von Durchleitungen 8 angeordnet ist.

Die Durchleitungen 8 sind im in Figur 1 gezeigten Ausführungsbeispiel in einer regelmäßigen Form angeordnet. Alternativ dazu ist auch eine freie oder willkürliche Anordnung denkbar. Ein Abstand zwischen jeweils zwei benachbarten Durchleitungen 8 muss dabei so groß gewählt werden, dass die Durchleitungen 8 einander nicht berühren, da dies zum Kurzschluss führen würde.

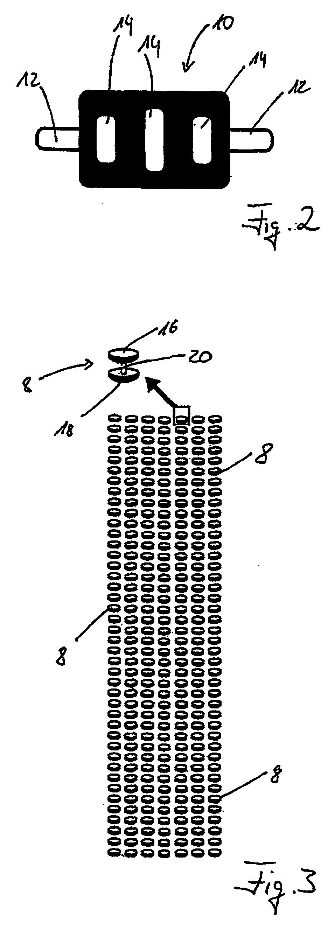

Figur 2 zeigt die schematische Darstellung einer Elektrode 10 für ein Prothesensystem gemäß einem Ausführungsbeispiel der vorliegenden Erfindung. Dabei ist die Elektrode 10 in Figur 2 von unten, also der am Liner anliegenden Seite dargestellt. Man erkennt auf der rechten und der linken Seite jeweils eine Positionierungshilfe 12, durch die die Elektrode 10 besser und einfacher positioniert werden kann. Zudem sind nicht gezeigte Zuleitungen vorgesehen, durch die die von der Elektrode 10 aufgenommenen elektrischen Signale weiter transportiert werden können. Die in Figur 2 gezeigte Elektrode 10 weist drei Kontaktflächen 14 auf, durch die elektrische Signale aufgenommen werden können. Diese sind im in Figur 2 gezeigten Ausführungsbeispiel hochkant nebeneinander ausgerichtet, wobei die mittlere Kontaktfläche 14 größer ausgebildet ist als die benachbarten Kontaktflächen 14. Hier sind natürlich auch andere Anordnungen und Anzahlen von Kontaktflächen 14 denkbar. Die gewählte Anzahl und Anordnung hängt insbesondere von den Funktionen des Prothesensystems ab, die über die abgenommenen elektrischen Signale gesteuert werden sollen.

Figur 3 zeigt die Anordnung von Durchleitungen 8, wie sie in Figur 1 im Bereich 6 des Liners 2 dargestellt ist. Im oberen Bereich der Figur 3 ist eine vergrößerte Darstellung einer Durchleitung 8 gezeigt. Die Durchleitung 8 verfügt über eine Außenkontaktfläche 16, mit der eine Kontaktfläche 14 einer Elektrode 10 in Kontakt kommen kann. Die Durchleitung 8 verfügt zudem über eine Innenkontaktfläche 18, mit der die Durchleitung 8 im angelegten Zustand des Liners 2 in Kontakt mit der Haut am Amputationsstumpf des Patienten kommen kann. Zwischen der Außenkontaktfläche 16 und der Innenkontaktfläche 18 befindet sich ein Durchleitungselement 20, das aus einem elektrisch leitenden Material besteht, und mit dem elektrische Signale von der Innenkontaktfläche 18 zur Außenkontaktfläche 16 und umgekehrt geleitet werden können.

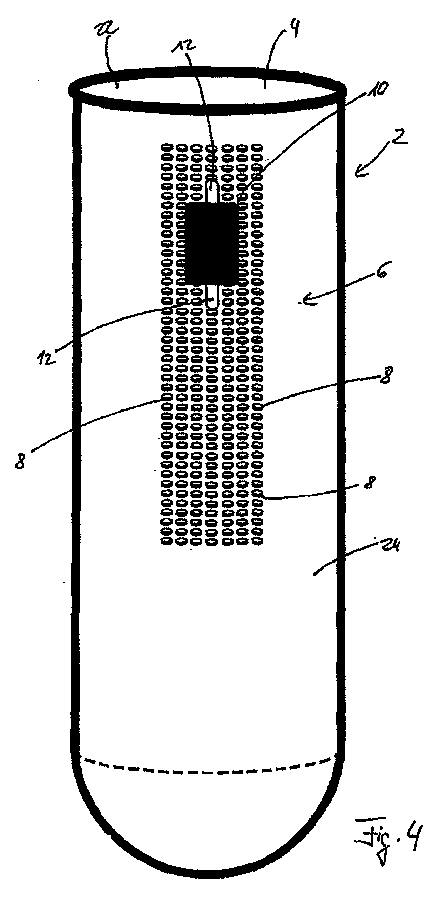

Figur 4 zeigt den Liner 2, der in einem Bereich 6 über eine Mehrzahl von Durchleitungen 8 verfügt. An diesem ist eine Elektrode 10 schematisch dargestellt, wie sie erfindungsgemäß an einer Schaftinnenfläche eines Prothesenschaftes angeordnet ist. Wird der Prothesenschaft über einen Amputationsstumpf gezogen, an dem vorher ein Liner 2 gemäß Figur 4 angeordnet ist, kommt die Elektrode 10, die an der Schaftinnenfläche des Prothesenschaftes angeordnet ist, beispielsweise in der in Figur 4 gezeigten Form mit einigen der Durchleitungen 8 in Kontakt.

Der Liner 2 verfügt über eine Innenfläche 22, mit der er mit dem Amputationsstumpf des Patienten in Kontakt kommt, und über eine Außenfläche 24, die der Innenfläche 22 abgewandt ist.

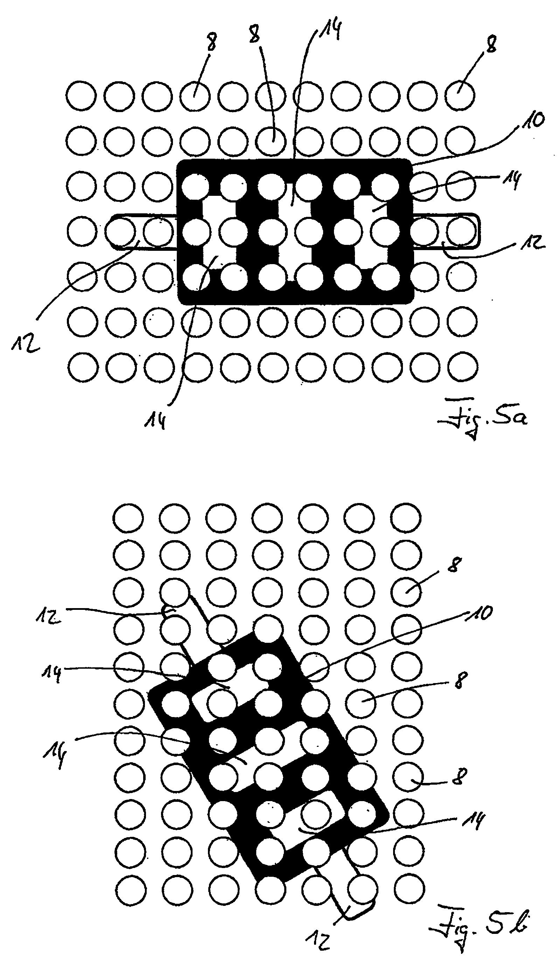

Die Figuren 5a und 5b zeigen schematisch mögliche Anordnungen einer Elektrode 10 relativ zu einer Mehrzahl von Durchleitungen 8. Man erkennt, dass jede der Kontaktflächen 14 in beiden in den Figuren 5a und 5b dargestellten Anordnungen mit wenigstens einer Durchleitung in Kontakt kommt, so dass elektrische Signale, die von einer Innenkontaktfläche 18 einer Durchleitung 8 zur Außenkontaktfläche 16 der Durchleitung 8 geleitet werden, von den Kontaktflächen 14 der Elektrode 10 aufgenommen. Auch die hier gezeigten Elektroden 10 sind mit Positionierungshilfen 12 ausgestaltet. Dabei ist es, wie in den Figuren 5a und 5b dargestellt, egal, in welcher Ausrichtung die Elektrode 10 relativ zur Anordnung der Durchleitungen 8 angeordnet wird. Dadurch, dass der Abstand zwischen zwei Durchleitungen 8 kleiner ist als die Kontaktfläche 14 kommt es immer zu einem Kontakt der Kontaktflächen 14 und der Außenkontaktfläche 16 der gezeigten Durchleitungen 8. Dadurch, dass der Bereich 6, in dem die Durchleitungen 8 angeordnet sind, größer sind als die Ausdehnung der Elektrode 10 und insbesondere die Ausdehnung der Kontaktflächen 14 der Elektrode 10, ist gewährleistet, dass auch ein Verschieben, Verrutschen oder Verdrehen des Liners 2 für die Abnahme der elektrischen Signale unschädlich ist. Es kommt in jedem dieser Fälle zu einem Kontakt, so dass myoelektrische Signale an der richtigen Stelle abgenommen, weitergeleitet und verarbeitet werden können.

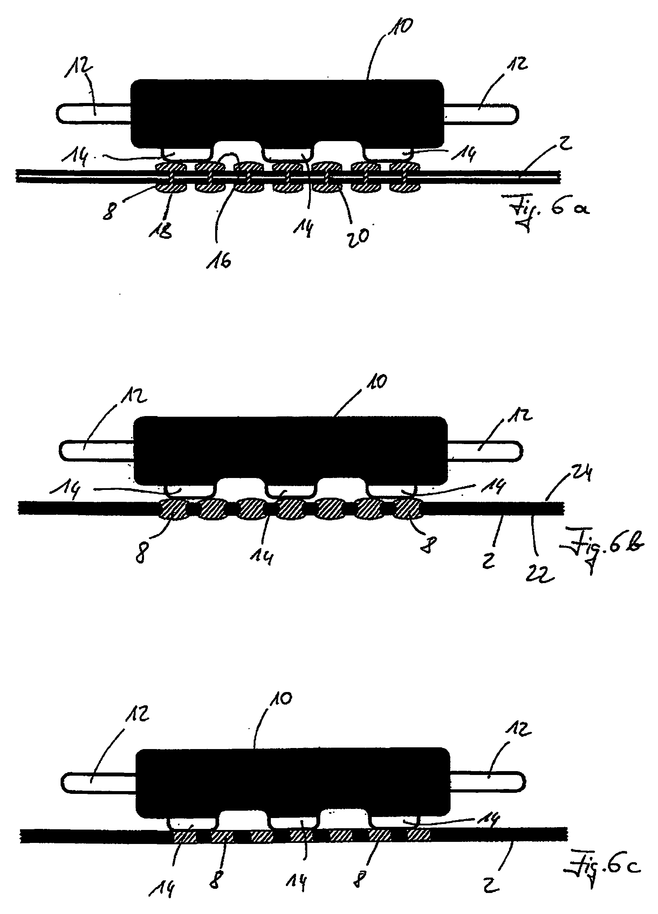

Die Figuren 6a, b und c zeigen die Anordnung einer Elektrode 10 relativ zu Durchleitungen 8 und dem Liner 2 in einer schematischen Schnitt- bzw. Seitenansicht. Man erkennt, dass die Elektrode 10 über drei Kontaktflächen 14 verfügt, die jeweils mit einer Außenkontaktfläche 16 einer Durchleitung 8 in Kontakt kommen. Insbesondere in Figur 6a ist deutlich gezeigt, dass über das Durchleitungselement 20 jede Außenkontaktfläche 16 mit einer ihr zugeordneten Innenkontaktfläche 18 der jeweiligen Durchleitung 8 in Verbindung steht. In den Figuren 6a und 6b sind die Durchleitungen 8 relativ zur Fläche des Liners 2 erhaben ausgebildet. Dies gilt sowohl für die Innenfläche 22 als auch für die Außenfläche 24 des Liners 2. Lediglich in Figur 6c sind die Innenfläche 22 und die Außenfläche 24 des Liners 2 eben und gleichmäßig ausgebildet. Jeweils an der Unterseite des Liners 2, an der sich die Innenfläche 22 befindet, kommt der Liner 2 mit der Haut am Amputationsstumpf des Patienten in Kontakt. Auf der gegenüber liegenden Außenfläche 24 wird, wie in den Figuren 6a bis c dargestellt, der Kontakt zu den Kontaktflächen 14 der Elektrode 10 hergestellt. Wenn die Innenkontaktfläche 18 und die Außenkontaktfläche 16 der Durchleitungen 8 eines Liners 2 erhaben ausgebildet sind, wie in den Figuren 6a und 6b gezeigt, wird der Kontakt zwischen der Außenkontaktfläche 16 und der Kontaktfläche 14 der Elektrode 10 vereinfacht. Bei einer Ausgestaltung gemäß Figur 6c, wo die Durchleitungen 8 einstückig und nicht erhaben ausgebildet sind, kommt es insbesondere nicht zu Druckstellen am Amputationsstumpf des Patienten.

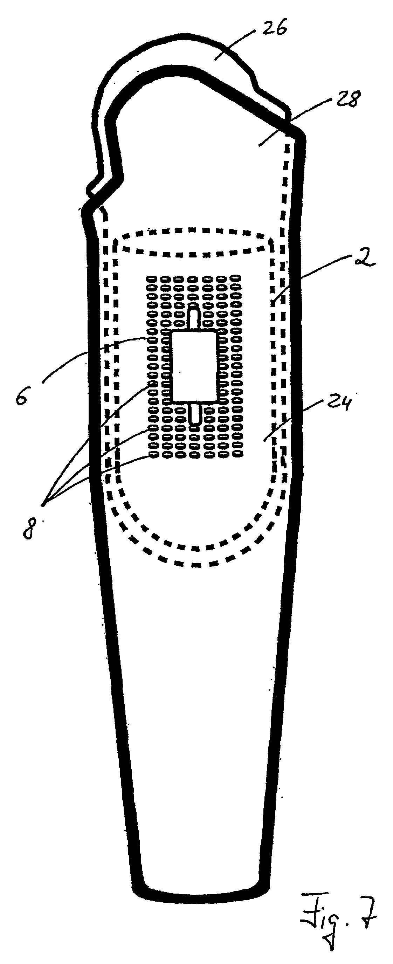

Figur 7 zeigt schematisch ein Prothesensystem gemäß einem Ausführungsbeispiel der vorliegenden Erfindung. An einem nicht gezeigten Amputationsstumpf ist zunächst der Liner 2 angeordnet. Darüber befindet sich ein Prothesenschaft, der einen Innenschaft 26 und einen darüber angeordneten Außenschaft 28 umfasst. Dabei wird vorzugsweise die Elektrode 10 an der Innenseite des Innenschafts 26 oder in den Innenschaft 26 integriert angeordnet. Wo diese in Figur 7 eigentlich durch den Außenschaft 28 verdeckt sind, sind sie durch gestrichelte Linien dargestellt. Auf der Außenfläche 24 des Liners 2 befindet sich ein Bereich 6 mit einer Mehrzahl von Durchleitungen 8, an denen schematisch eine Elektrode 10 angeordnet ist. Die Elektrode 10 ist dabei am Innenschaft 26 angeordnet, so dass eine exakte Ausrichtung der Elektrode 10 relativ zum Amputationsstumpf einfach möglich ist.

Selbst wenn der Liner 2 in der in Figur 7 gezeigten Konfiguration gedreht oder verschoben wird, kommt es immer noch zum Kontakt zwischen der Haut des Patienten am Amputationsstumpf, wenigstens einer Durchleitung 8 und der Elektrode 10 mit ihren Kontaktflächen 14, die in Figur 7 nicht gezeigt sind. Die Position, an der die elektrischen Signale vom Amputationsstumpf abgenommen werden, wird folglich lediglich von den Kontaktflächen 14 der Elektrode bestimmt. Die exakte Position des Liners 2 ist dabei nicht wichtig, so dass ein Verschieben oder Verdrehen des Liners 2 für die Abnahme insbesondere myoelektrischer Signale unschädlich ist.

### Bezugszeichenliste

- 2: Liner
- 4: Öffnung
- 6: Bereich
- 8: Durchleitung
- 10: Elektrode
- 12: Positionierungshilfe
- 14: Kontaktfläche
- 16: Außenkontaktfläche
- 18: Innenkontaktfläche
- 20: Durchleitungselement
- 22: Innenfläche
- 24: Außenfläche
- 26: Innenschaft
- 28: Außenschaft

## Patentansprüche

1. Prothesensystem mit
- einem Liner (2) aus einem elektrisch nicht leitenden Linermaterial, der vorgesehen ist, über einen Amputationsstumpf gezogen zu werden, und der eine zur Anlage an dem Amputationsstumpf vorgesehene Innenfläche (22) und eine der Innenfläche (22) abgewandte Außenfläche (24) aufweist,
- wenigstens einer Elektrode (10), die wenigstens eine Kontaktfläche (14) aufweist, und
- einem Prothesenschaft, der vorgesehen ist, an dem Amputationsstumpf angeordnet zu werden, nachdem über diesen der Liner (2) gezogen wurde, so dass eine Schaftinnenfläche der Außenfläche (24) des Liners (2) zugewandt ist, wobei
- die wenigstens eine Elektrode (10) an der Außenfläche (24) des Liners (2) oder an der Schaftinnenfläche des Prothesenschaftes angeordnet ist,
- in wenigstens einem Bereich (6) des Liners (2) eine Mehrzahl von Durchleitungen (8) von der Innenfläche (22) zu der Außenfläche (24) aus einem elektrisch leitenden Material in dem Linermaterial angeordnet sind, und
- die wenigstens eine Kontaktfläche (14) der wenigstens einen Elektrode (10) vorgesehen ist, mit wenigstens einer Durchleitung (8) in Kontakt zu kommen, wenn der Prothesenschaft an dem Amputationsstumpf (26) angeordnet wird, nachdem über diesen der Liner (2) gezogen wurde,
**dadurch gekennzeichnet, dass**
- der wenigstens eine Bereich (6) größer ist als, bevorzugt wenigstens doppelt so groß ist wie die wenigstens eine Kontaktfläche (14) der wenigstens einen Elektrode (10), sodass ein Verdrehen oder Verschieben des Liners (2) relativ zu dem Amputationsstumpf für eine Qualität abgenommener elektrischer Signale unschädlich ist.

2. Prothesensystem nach Anspruch 1, **dadurch gekennzeichnet, dass** an der Schaftinnenfläche mehrere Elektroden (10) angeordnet sind und der Liner (2) mehrere Bereiche (6) mit einer Mehrzahl an Durchleitungen (8) vorgesehen aufweist.

3. Prothesensystem nach Anspruch 1 oder 2, **dadurch gekennzeichnet, dass** die Mehrzahl an Durchleitungen (8) einstückig mit dem Liner (2) ausgebildet sind.

4. Prothesensystem nach Anspruch 1 oder 2, **dadurch gekennzeichnet, dass** die Mehrzahl an Durchleitungen (8) in das Linermaterial eingebrachte Nieten sind.

5. Prothesensystem nach einem der vorstehenden Ansprüche, **dadurch gekennzeichnet, dass** die wenigstens eine Elektrode (10) verschieblich an der Außenseite (24) des Liners (2) oder an der Schaftinnenfläche angeordnet ist.

6. Prothesensystem nach einem der vorstehenden Ansprüche, **dadurch gekennzeichnet, dass** die wenigstens eine Elektrode (10) mehrere Kontaktflächen (14) aufweist.

7. Prothesensystem nach einem der vorstehenden Ansprüche, **dadurch gekennzeichnet, dass** eine Außenkontaktfläche (16) jeder Durchleitung (8) an der Außenseite (24) des Liners (2) für den Kontakt mit der wenigstens einen Kontaktfläche (14) der wenigstens einen Elektrode (10) größer oder gleich 1 mm2 ist.

8. Prothesensystem nach einem der vorstehenden Ansprüche, **dadurch gekennzeichnet, dass** das nichtleitende Linermaterial ein hydrophobes Material ist.

9. Prothesensystem nach einem der nachstehenden Ansprüche, **dadurch gekennzeichnet, dass** das elektrisch leitende Material der Durchleitungen (8) ein hydrophiles Material ist.

10. Prothesensystem nach einem der vorstehenden Ansprüche, **dadurch gekennzeichnet, dass** eine Innenkontaktfläche (18) jeder Durchleitung (8) an der Innenfläche (22) des Liners (2) genau so groß ist wie eine Außenkontaktfläche (16) jeder Durchleitung (8) an der Außenfläche (24) des Liners (2).

## Claims

1. Prosthesis system with
- a liner (2) made of an electrically nonconducting liner material, which is provided to be pulled over an amputation stump and which has an inner face (22) provided to rest against the amputation stump and an outer face (24) facing away from the inner face (22),
- at least one electrode (10) which has at least one contact face (14), and
- a prosthesis socket, which is provided to be arranged on the amputation stump after the liner (2) was pulled thereover such that a socket inner face faces the outer face (24) of the liner (2),
**wherein**
- the at least one electrode (10) is arranged on the outer face (24) of the liner (2) or on the socket inner face of the prosthesis socket,
- a plurality of feedthroughs (8) which run from the inner face (22) to the outer face (24) and are made of an electrically conducting material in the liner material are arranged in at least one region (6) of the liner (2)
and
- the at least one contact face (14) of the at least one electrode (10) is provided to come into contact with at least one feedthrough (8) when the prosthesis socket is arranged on the amputation stump (26) after the liner (2) was pulled over the latter,
**characterized in that** the at least one region (6) is greater than the at least one contact face (14) of the at least one electrode (10), preferably at least twice the size thereof, such that a twist or shift of the liner (2) relative to the amputation stump is harmless for a quality of picked-up electric signals.

2. Prosthesis system according to Claim 1, **characterized in that** a plurality of electrodes (10) are arranged on the socket inner face and the liner (2) has a plurality of regions (6) where a plurality of feedthroughs (8) are provided.

3. Prosthesis system according to Claim 1 or 2, **characterized in that** the plurality of feedthroughs (8) have an integral design with the liner (2).

4. Prosthesis system according to Claim 1 or 2, **characterized in that** the plurality of feedthroughs (8) are rivets introduced into the liner material.

5. Prosthesis system according to one of the preceding claims, **characterized in that** the at least one electrode (10) is displaceably arranged on the outside (24) of the liner (2) or on the socket inner face.

6. Prosthesis system according to one of the preceding claims, **characterized in that** the at least one electrode (10) has a plurality of contact faces (14).

7. Prosthesis system according to one of the preceding claims, **characterized in that** an outer contact face (16) of each feedthrough (8) on the outside (24) of the liner (2) is greater than or equal to 1 mm² for the contact with the at least one contact face (14) of the at least one electrode (10).

8. Prosthesis system according to one of the preceding claims, **characterized in that** the nonconducting liner material is a hydrophobic material.

9. Prosthesis system according to one of the subsequent claims, **characterized in that** the electrically conducting material of the feedthroughs (8) is a hydrophilic material.

10. Prosthesis system according to one of the preceding claims, **characterized in that** an inner contact face (18) of each feedthrough (8) on the inner face (22) of the liner (2) has exactly the same size as an outer contact face (16) of each feedthrough (8) on the outer face (24) of the liner (2).

## Revendications

1. Système de prothèse comportant
- une gaine (2) constituée d'un matériau de gaine non conducteur de l'électricité et prévue pour être enfilée par-dessus un moignon d'amputation et qui présente une surface intérieure (22) prévue pour venir en appui contre le moignon et une surface extérieure (24) détournée de la surface intérieure (22),
- au moins une électrode (10) qui présente au moins une surface de contact (14), et
- une emboîture de prothèse prévue pour être agencée sur le moignon après avoir enfilé la gaine (2) par-dessus celui-ci, de sorte qu'une surface intérieure de l'emboîture est tournée vers la surface extérieure (24) de la gaine (2),
dans lequel
- ladite au moins une électrode (10) est agencée sur la surface extérieure (24) de la gaine (2) ou sur la surface intérieure de l'emboîture de prothèse,
- dans au moins une zone (6) de la gaine (2) est prévue une pluralité de lignes (8) traversantes de la surface intérieure (22) vers la surface extérieure (24) constituées d'un matériau électriquement conducteur et disposées dans le matériau de la gaine,
et
- ladite au moins une surface de contact (14) de ladite au moins une électrode (10) est prévue pour venir en contact avec au moins une ligne traversante (8) lorsque l'emboîture de prothèse est agencée sur le moignon (26) après avoir enfilé la gaine (2) par-dessus celui-ci, **caractérisé en ce que**
- ladite au moins une zone (6) est plus grande, de préférence au moins deux fois plus grande que ladite au moins une surface de contact (14) de ladite au moins une électrode (10), de sorte qu'une rotation ou une translation de la gaine (2) par rapport au moignon est sans effet négatif sur une qualité des signaux électriques captés.

2. Système de prothèse selon la revendication 1, **caractérisé en ce que** plusieurs électrodes (10) sont agencées sur la surface intérieure de l'emboîture, et la gaine (2) comprend plusieurs zones (6) pourvues d'une pluralité de lignes traversantes (8).

3. Système de prothèse selon la revendication 1 ou 2, **caractérisé en ce que** la pluralité de lignes traversantes (8) sont réalisées d'un seul tenant avec la gaine (2).

4. Système de prothèse selon la revendication 1 ou 2, **caractérisé en ce que** la pluralité des lignes traversantes (8) sont des rivets intégrés dans le matériau de la gaine.

5. Système de prothèse selon l'une des revendications précédentes, **caractérisé en ce que** ladite au moins une électrode (10) est agencée de façon mobile en translation sur la face extérieure (24) de la gaine (2) ou sur la surface intérieure de l'emboîture.

6. Système de prothèse selon l'une des revendications précédentes, **caractérisé en ce que** ladite au moins une électrode (10) comprend plusieurs surfaces de contact (14).

7. Système de prothèse selon l'une des revendications précédentes, **caractérisé en ce qu'**une surface de contact extérieure (16) de chaque ligne traversante (8) prévue sur la face extérieure (24) de la gaine (2) pour le contact avec ladite au moins une surface de contact (14) de ladite au moins une électrode (10) est supérieure ou égale à 1 mm².

8. Système de prothèse selon l'une des revendications précédentes, **caractérisé en ce que** le matériau non conducteur de la gaine est un matériau hydrophobe.

9. Système de prothèse selon l'une des revendications précédentes, **caractérisé en ce que** le matériau conducteur de l'électricité des lignes traversantes (8) est un matériau hydrophile.

10. Système de prothèse selon l'une des revendications précédentes, **caractérisé en ce qu'**une surface de contact intérieure (18) de chaque ligne traversante (8) sur la surface intérieure (22) de la gaine (2) est exactement aussi grande qu'une surface de contact extérieure (16) de chaque ligne traversante (8) sur la surface extérieure (24) de la gaine (2).
